(19) Europäisches Patentamt
European Patent Office
Office européen des brevets

(11) Veröffentlichungsnummer : **0 047 714**
**B1**

(12) # EUROPÄISCHE PATENTSCHRIFT

(45) Veröffentlichungstag der Patentschrift : **30.10.85**

(51) Int. Cl.⁴ : C 08 L 33/14, C 08 L 33/26, A 61 K 7/06, C 11 D 3/37

(21) Anmeldenummer : **81810306.1**

(22) Anmeldetag : **27.07.81**

(54) **Gemische aus quaternären, polymeren, hochmolekularen Ammoniumsalzen auf Acrylbasis und Tensiden, deren Herstellung und Verwendung in kosmetischen Mitteln.**

(30) Priorität : **05.09.80 CH 6688/80**

(43) Veröffentlichungstag der Anmeldung : **17.03.82 Patentblatt 82/11**

(45) Bekanntmachung des Hinweises auf die Patenterteilung : **30.10.85 Patentblatt 85/44**

(84) Benannte Vertragsstaaten : **BE CH DE FR GB IT LI NL**

(56) Entgegenhaltungen :
**GB-A- 1 073 947**
**GB-A- 1 195 158**
**GB-A- 1 342 176**
**GB-A- 2 027 045**
**US-A- 3 313 734**
**US-A- 3 996 146**
**US-A- 4 009 256**

**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Postfach**
**CH-4002 Basel (CH)**

(72) Erfinder : **Strasilla, Dieter, Dr.**
**Efringerstrasse 26**
**D-7858 Weil am Rhein (DE)**
Erfinder : **Moldovanyi, Laszlo, Dr.**
**Austrasse 78**
**CH-4051 Basel (CH)**
Erfinder : **Fearnley, Charles, Dr.**
**Wettsteinanlage 50**
**CH-4125 Riehen (CH)**
Erfinder : **Meindl, Hubert, Dr.**
**Hungerbachweg 58**
**CH-4125 Riehen (CH)**

**EP 0 047 714 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Gemische aus quaternären, polymeren, hochmolekularen Ammoniumsalzen auf Acrylbasis und nicht-ionischen Tensiden oder Tensiden mit intramolekular je einer positiven und negativen Ladung, wobei anionische Tenside ausgeschlossen sind.

Im Gegensatz hierzu offenbart US-A-3 313 734 Gemische aus quaternären, polymeren, hochmolekularen Ammoniumsalzen und anionischen, nicht-ionischen bzw. ampholytischen Tensiden mit intramolekular einer positiven und einer oder zwei negativen Ladungen, wobei anionische Tenside stets mitverwendet werden.

Gegenstand der vorliegenden Erfindung sind somit wässerige Gemische aus polymeren, quaternären Ammoniumsalzen und Tensiden, die dadurch gekennzeichnet sind, dass sie

a) mindestens ein in wässrigen Tensidsystemen lösliches oder mikroemulgierbares Ammoniumsalz, das eine Molekulargewichtsverteilung von $10^4$ bis $10^9$ aufweist, wobei das Molekulargewicht von mindestens 5 Gewichtsprozent des copolymeren Salzes $10^7$ bis $10^9$ beträgt und das Salz wiederkehrende Strukturelemente der Formel

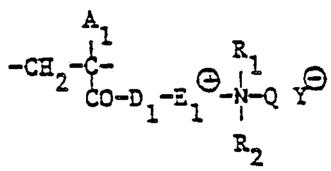

(1)

und gegebenenfalls in beliebiger Reihenfolge mindestens eines der wiederkehrenden Strukturelemente der Formeln

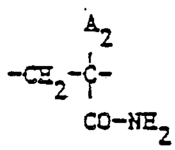

(2)

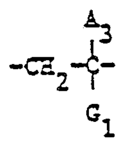

und (3)

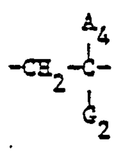

(4)

aufweist, worin $A_1$, $A_2$, $A_3$ und $A_4$ je Wasserstoff oder Methyl, $G_1$ und $G_2$ voneinander verschieden sind und je —CN, —COOH oder

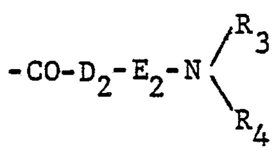

$D_1$ und $D_2$ je Sauerstoff oder —NH—, $E_1$ und $E_2$ je Alkylen mit 1 bis 4 Kohlenstoffatomen, das gegebenenfalls durch Hydroxyl substituiert ist, $R_1$, $R_2$, $R_3$ und $R_4$ je Methyl oder Aethyl, Q Alkyl, Hydroxyalkyl mit 1 bis 4 Kohlenstoffatomen oder Benzyl und $Y^{\ominus}$ ein Halogenid-, Alkylsulfat- oder Alkylphosphonatanion mit 1 bis 4 Kohlenstoffatomen im Alkylrest bedeuten, und

b) mindestens ein nicht-ionisches Tensid oder ein Tensid mit intramolekular je einer positiven und negativen Ladung unter Ausschluss von anionischen Tensiden in einem Gewichtsverhältnis (a) : (b) von (1) : (2 bis 400) enthalten.

Weitere Gegenstände der Erfindung sind das Herstellungsverfahren für die vorstehend angegebenen Gemische, deren Verwendung in kosmetischen Mitteln, die kosmetischen Mittel (Haarkosmetik), welche die erfindungsgemässen Gemische enthalten und deren Applikationsverfahren, insbesondere Verfahren zum Behandeln von Haar sowie nach den Applikationsverfahren behandeltes Haar, z. B. in Form von Perücken.

Die in den erfindungsgemässen Gemischen als Komponente (a) eingesetzten, polymeren Ammoniumsalze zeichnen sich dadurch aus, dass sie durch Wasser-in-Oel Emulsionspolymerisation oder Lösungspolymerisation eines quaternären Ammoniumsalzes der Acrylsäurereihe und gegebenenfalls mindestens eines weiteren Comonomers auf Acrylbasis erhältlich sind.

Durch die Wasser-in-Oel Emulsionspolymerisation, auch inverse Emulsionspolymerisation genannt, bzw. die Lösungspolymerisation, erreicht man den hohen Molekulargewichtsbereich der erfindungsgemäss eingesetzten Polymerisate von $10^7$ bis $10^9$, deren Anteil vorzugsweise 5 bis 60 und insbesondere 20 bis 50 Gewichtsprozent der Copolymerisate innerhalb der breiten Molekulargewichtsverteilung von $10^4$ bis $10^9$ ausmacht. Besonders bevorzugte Polymere weisen 30-45 Gew.% Anteile im Molgewichtsbereich $10^7$ bis $10^9$ und weniger als 15 Gew.% Anteile im Molgewichtsbereich kleiner als $10^5$ auf.

Der Anteil an Strukturelementen der Formel (1) in den Polymerisaten, auch Quatgehalt genannt, bildet neben der Molekulargewichtsverteilung ein weiteres, wesentliches Kennzeichen der eingesetzten Ammoniumsalze, welche durchschnittlich etwa 5 bis 100, vorzugsweise 6 bzw. 9 bis 40 und insbesondere 10 bis 30 Mol% Strukturelemente der Formel (1), durchschnittlich etwa 0 bis 95, vorzugsweise 10 bis 95 und insbesondere 50 bis 90 Mol% Strukturelemente der Formel (2) und durchschnittlich etwa 0 bis 10, vorzugsweise insgesamt 1 bis 8 Mol% Strukturelemente der Formel (3) und gegebenenfalls (4), d. h. (3) und/oder (4) und insbesondere je 1 bis 4 Mol% Strukturelemente der Formeln (3) und (4) enthalten. Ammoniumsalze mit einem Quatgehalt, d. h. ein Gehalt an Strukturelemente der Formel (1) von 100 % aufweisen, stellen Homopolymerisate dar. Der Quatgehalt von 100 % ist aber als Idealwert anzusehen, da durch Hydrolyse der Strukturelemente der Formel (1) die Homopolymerisate stets Spuren (z. B. 0,01 bis 0,5 Gewichtsprozent) an Strukturelemente der Formel (4), worin $G_2$ —COOH bedeuten, enthalten. Copolymerisate, die mindestens eines der Strukturelemente (2), (3) und/oder (4) aufweisen, sind den Homopolymerisaten gegenüber als Komponente (a) der erfindungsgemässen Gemische bevorzugt.

Als bevorzugte Bedeutungen für die Symbole der Formel (1) gelten für $A_1$ Methyl, für $D_1$ Sauerstoff, für $E_1$ unsubstituiertes n-Propyl oder insbesondere unsubstituiertes Aethylen, für $R_1$ und $R_2$ Methyl und für Q unsubstituiertes n-Propyl, vorzugsweise Aethyl oder insbesondere Methyl. $A_2$ in Formel (2) steht vorzugsweise für Wasserstoff. Falls Strukturelemente der Formel (3) für sich allein, d. h. in Abwesenheit von Strukturelementen der Formel (4), vorhanden sind, gelten als bevorzugte Bedeutungen für die Symbole der Formel (3) Methyl für $A_3$ und

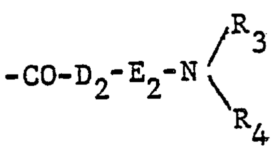

für $G_1$, wobei $D_2$, $E_2$, $R_3$ und $R_4$ die gleichen bevorzugten Bedeutungen haben wie für $D_1$, $E_1$, $R_1$ und $R_2$ vorstehend angegeben. Falls Strukturelemente der Formel (4) zusätzlich zu den Strukturelementen der Formel (3) vorhanden sind, bedeuten in Formel (4) $A_4$ vorzugsweise Wasserstoff und $G_2$ vorzugsweise —CN oder $A_4$ insbesondere Methyl und $G_2$ insbesondere —COOH.

Als nicht-ionische Tenside für die Komponente (b) in den erfindungsgemässen Gemischen kommen äthoxylierte/propoxylierte Fettalkohole, Fettamine, Fettsäuren, Fettsäureamide, Alkylphenole oder Kohlenhydrate, worin die endständigen Hydroxylgruppen gegebenenfalls veräthert sind und insbesondere Alkyläther mit 1 bis 20 Kohlenstoffatomen im Aetherteil vorliegen, Addukte (Blockcopolymere) aus Aethylen- und Propylenoxyd, Phosphorsäurepolyglykolester oder Aminoxyde, die vorzugsweise einen Fettrest aufweisen, in Betracht.

Geeignet sind ferner auch Fettsäureester von 3- bis 6-wertigen Alkoholen (Glycerin, Pentaerythrit, Sorbit, Sorbitan) oder von Mono- und Disacchariden (Saccharose).

Die als nicht-ionische Tenside verwendeten, äthoxylierten Fettsäuren, Fettalkohole, Fettsäureamide, Alkylphenole oder Kohlenhydrate entsprechen vorzugsweise einer der Formeln

$$H-(O-CH_2-CH_2)_p-OOC-T_1 \quad (5)$$

$$H-(O-CH_2-CH_2)_p-NH-CO-T_1, \quad (6)$$

$$H-(O-CH_2-CH_2)_p-O-T_2, \quad (7)$$

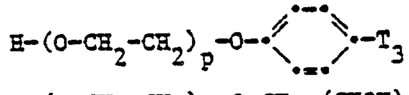 oder (8)

$$H-(O-CH_2-CH_2)_p-O-CH_2-(CHOH)_s-CHO \quad (9)$$

worin $T_1$ Alkyl oder Alkenyl mit 7 bis 21, vorzugsweise 11 bis 17 Kohlenstoffatomen, $T_2$ Alkyl oder Alkenyl mit 8 bis 22, vorzugsweise 12 bis 18 Kohlenstoffatomen, $T_3$ Alkyl mit 6 bis 14, vorzugsweise 8 bis 12 Kohlenstoffatomen, p eine ganze Zahl von 1 bis 50, vorzugsweise 1 bis 20 und s 3 oder vorzugsweise 4 bedeuten.

In Formel (5) leiten sich die Reste $T_1COO$— von den entsprechenden gesättigten oder ungesättigten Fettsäuren mit 8 bis 22, vorzugsweise 12 bis 18 Kohlenstoffatomen ab. Als Beispiele der entsprechenden

Fettsäuren seien Capryl-, Caprin-, Arachin- und Behensäure, insbesondere Laurin-, Myristin-, Palmitin- und Stearinsäure oder Myristolein-, Palmitolein-, Elaeostearin, Clupanodonsäure, insbesondere Oel-, Elaidin-, Eruka-, Linol- und Linolensäure genannt. Alkyl- und Alkenylreste für $T_1$, die sich von technischen Gemischen der genannten gesättigten und/oder ungesättigten Fettsäuren ableiten, sind besonders bevorzugt. Die Fettalkohol- und Fettsäureamidreste $T_1$ —CO—NH— und $T_2$ —O— in den Formeln (6) und (7) leiten sich vorzugsweise von den entsprechenden, vorstehend erwähnten Fettsäuren ab.

Bevorzugte Alkylreste für $T_2$ in Formel (8) sind z. B. Alkylreste mit 6 bis 12 Kohlenstoffatomen wie Hexyl, i-Hexyl, Heptyl, i-Octyl, Dodecyl, ferner Alkylreste mit 12 bis 14 Kohlenstoffatomen, die sich von dimerisierten Olefinen mit je 6 oder 7 Kohlenstoffatomen ableiten.

Als äthoxylierte Kohlenhydrate kommen je nach dem Wert von s in Formel (9) vor allem äthoxylierte Pentosen und insbesondere Hexosen, z. B. äthoxylierte Glukose, in Betracht.

Gegebenenfalls können die äthoxylierten Tenside der Formeln (5) bis (9) auch in verätherter Form (Alkyläther mit 1 bis 20 Kohlenstoffatomen im Aetherteil) vorliegen.

Unter den äthoxylierten, nicht-ionischen Tensiden der Formel (5) bis (9) sind die äthoxylierten Fettsäuren und Fettalkohole der Formeln (5) und (7) und insbesondere die äthoxylierten Alkylphenole der Formel (8) bevorzugt. Als spezifisches Beispiel solcher äthoxylierten Alkylphenole sei u. a. das Tensid der Formel

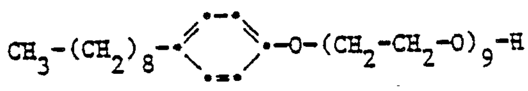 (10)

genannt.

Als nicht-ionisches Tensid für die Komponente (b) des erfindungsgemässen Gemisches sind ebenfalls handelsübliche Produkte aus Aethylenoxyd und Propylenoxyd bevorzugt, die durch Addition von Aethylenoxyd an ein Kondensationsprodukt aus Propylenoxyd mit Propylenglykol erhältlich sind und Molekulargewichte von etwa 1 000 bis etwa 15 000 aufweisen. Solche Addukte stellen Blockcopolymerisate dar, die der wahrscheinlichen Formel

$$HO-(CH_2-CH_2-O)_x-(CH_2-\underset{CH_3}{CH}-O)_y-(CH_2-CH_2-O)_z-H \qquad (11)$$

entsprechen, worin x, y und z gleiche oder voneinander verschiedene, ganze Zahlen bedeuten. Die Werte für x, y und z hängen vom Molekulargewicht des Copolymerisates ab und stellen nur Durchschnittswerte dar. Besonders bevorzugt sind Copolymerisate mit durchschnittlichen Molekulargewichten von 2 000 bis 8 000, worin die Durchschnittswerte von x und z je zwischen 2 und 60 und von y zwischen 20 und 80 schwanken und somit der wahrscheinlichen Formel

$$HO-(CH_2-CH_2-O)_{2-60}-(CH_2-\underset{CH_3}{CH}-O)_{20-80}-(CH_2-CH_2-O)_{2-60}-H \qquad (12)$$

entsprechen.

Als nicht-ionisches Tensid für die Komponente (b) kommen auch Phosphorsäurepolyglykolester und Aminoxyde, vor allem solche mit Fettresten, in Frage, wobei die Phosphorsäurepolyglykolester vorzugsweise der Formel

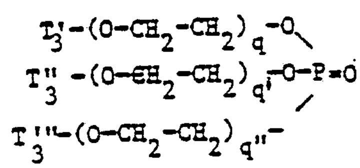 (13)

entsprechen, worin $T_3'$ $T_3''$ und $T_3'''$ voneinander verschieden oder vorzugsweise gleich sind und jeweils Alkyl mit 6 bis 14 Kohlenstoffatomen bedeuten, und q, q' und q'' voneinander verschieden oder vorzugsweise gleich sind und eine ganze Zahl von je 6 bis 12 bedeuten, und Aminoxyde vorzugsweise einer der Formeln

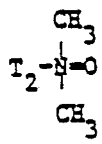 und insbesondere (14)

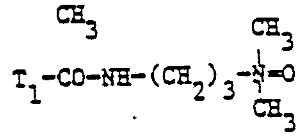 (15)

entsprechen, worin $T_1$ und $T_2$ die angegebenen Bedeutungen haben.

Geeignet sind ferner auch Fettsäureester von 3 bis 6-wertigen Alkoholen mit 3 bis 6 Kohlenstoffatomen oder von Mono- oder Disacchariden (Saccharose). Die Fettsäurereste leiten sich z. B. von gesättigten oder ungesättigten Fettsäuren mit vorzugsweise 12 bis 18 Kohlenstoffatomen (Laurin-, Palmitin-, Stearin-, Oelsäure) ab. Genannt seien insbesondere die Sorbitan-Fettsäureester.

Die nicht-ionischen Tenside der Formeln (5) bis (12), vor allem (5), (7), (8) und (11) und insbesondere (10) und (12), sind den nicht-ionischen Tensiden der Formeln (13) bis (15) gegenüber bevorzugt.

Als Komponente (b) kommen auch Tenside mit intramolekular je einer positiven und negativen Ladung in Betracht, welchen im allgemeinen gegenüber den nicht-ionischen Tensiden der angegebenen Art der orzug gegeben wird. Bei diesen Tensiden handelt es sich vor allem um Betaine oder Sulfobetaine, die sich von Imidazolinderivaten oder offenkettigen aliphatischen Aminen ableiten.

Die als Komponente (b) erfindungsgemäss eingesetzten, sich von Imidazolinderivaten ableitenden Betaine oder Sulfobetaine entsprechen vorzugsweise einer der Formeln

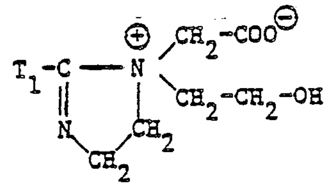

(16)

oder

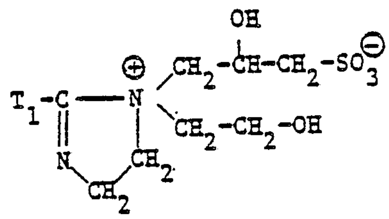

(17)

worin $T_1$ die angegebenen Bedeutungen hat.

Als Beispiele spezifischer Vertreter solcher Imidazoliniumbetaine oder -sulfobetaine seien u. a. die Tenside, bzw. technischen Tensidgemische der Formeln

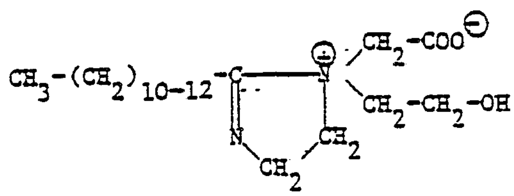

(18)

und

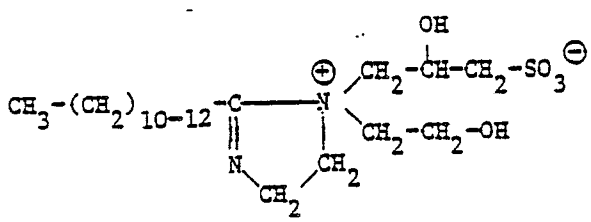

(19)

genannt.

Die erfindungsgemäss eingesetzten, sich von einem offenkettigen aliphatischen Amin ableitenden Betainderivate entsprechen vorzugsweise einer der Formeln

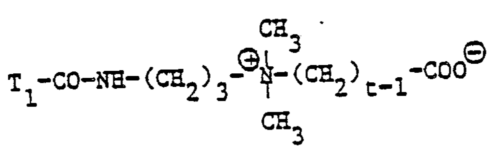

(20)

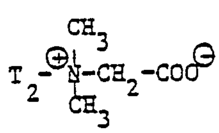

(21)

$$T_2-\overset{\oplus}{N}H_2-CH_2-(CH_2)_{t-1}-COO^{\ominus} \quad (22)$$

$$T_2-(NH-CH_2-CH_2)_{t-1}-NH-CH_2-CH_2-\overset{\oplus}{N}H_2-CH_2-COO^{\ominus} \quad (23)$$

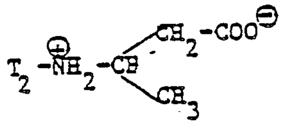

(24)

oder

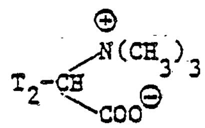

(25)

worin $T_1$ die angegebenen Bedeutungen hat, $T_2$ Alkyl oder Alkenyl mit 8 bis 22, vorzugsweise 12 bis 18 Kohlenstoffatomen und t 1 oder 2 bedeuten.

Als Beispiele spezifischer Vertreter solcher Betainderivate seien u. a. die Tenside bzw. technischen Tensidgemische der Formeln

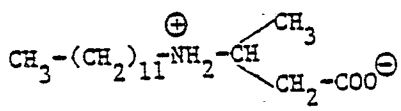

(26)

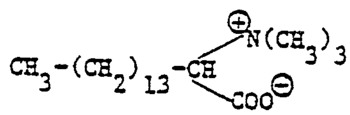

(27)

insbesondere

$$CH_3-(CH_2)_{11-13}-\overset{\oplus}{N}H_2-(CH_2)_2-COO^{\ominus} \quad (28)$$

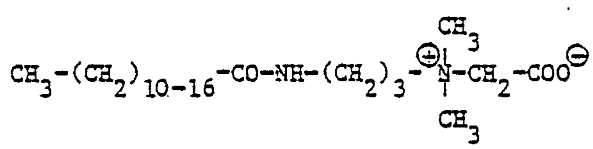

(29)

$$CH_3-(CH_2)_{10-12}-CH_2-\overset{\oplus}{N}H_2-(CH_2)_2COO^{\ominus} \quad (30)$$

und

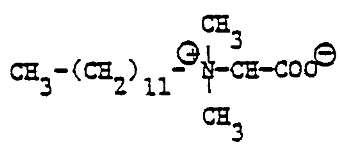

(31)

genannt.

Die erfindungsgemäss eingesetzten Sulfobetainderivate, die sich von einem aliphatischen, offenkettigen Amin ableiten, entsprechen vorzugsweise einer der Formeln

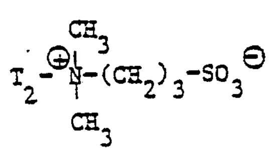

(32)

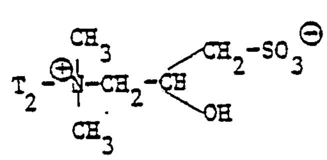

(33)

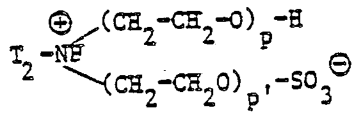 (34)

oder

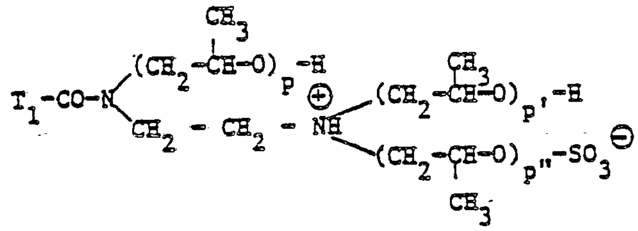 (35)

worin $T_1$ und $T_2$ die angegebenen Bedeutungen und p' und p" die für p angegebenen Bedeutungen haben, wobei p, p' und p" gleich oder voneinander verschieden sind.

Als Beispiele spezifischer Vertreter solcher Sulfobetainderivate seien u. a. die Tenside der Formeln

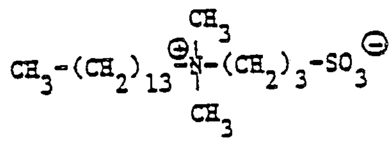 (36)

und

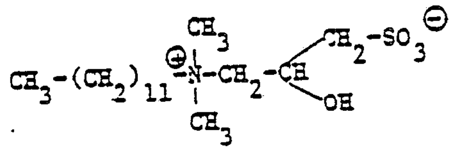 (37)

genannt.

Als Komponente (b) des erfindungsgemässen Gemisches stehen nicht-ionische Tenside der Formeln (12), vor allem (10) und insbesondere Tenside mit intramolekular je einer positiven und negativen Ladung der Formeln (18), (19) und (28) bis (31) im Vordergrund des Interesses.

Die erfindungsgemässen Gemische enthalten die Komponenten (a) und (b) in einem Gewichtsverhältnis (a) : (b), das vorzugsweise (1) : (4 bis 100) und insbesondere (1) : (10 bis 50) beträgt.

Zur Herstellung der erfindungsgemässen Gemische wird z. B. so verfahren, dass man ein Monomer der Formel

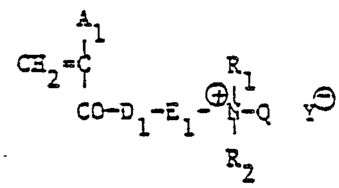 (38)

und gegebenenfalls mindestens ein Comonomer einer der Formeln

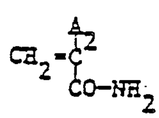 (39)

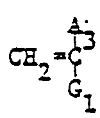 (40)

und

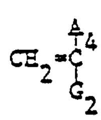 (41)

worin $A_1$, $A_2$, $A_3$, $A_4$, $D_1$, $E_1$, $G_1$, $G_2$, $R_1$, $R_2$, Q und $Y^{\ominus}$ die angegebenen Bedeutungen haben, durch Wasser-in-Oel Emulsionspolymerisation in Gegenwart eines Wasser-in-Oel Emulgators, gegebenenfalls eines Emulsionsstabilisators und eines Polymerisationsinitiators copolymerisiert, das Polymerisat mit einem in der Regel mit Oel und Wasser mischbaren Lösungsmittel ausfällt und anschliessend trocknet, wobei das Herstellungsverfahren dadurch gekennzeichnet ist, dass man das so erhaltene Polymerisat als Komponente (a) einsetzt und mit mindestens einem nicht-ionischen Tensid oder einem Tensid mit intramolekular je einer positiven und negativen Ladung als Komponente (b) in wässrigem Medium bei Raumtemperatur und einem pH-Wert von 5 bis 9 vermischt. Arbeitet man nach der Methode der Lösungspolymerisation, so ist der Einsatz von Emulgatoren und Emulsionsstabilisatoren nicht nötig. Als Lösungsmittel dient in der Regel Wasser.

Bei der Wasser-in-Oel Emulsionspolymerisation werden für die Oelphase hydrophobe, organische Flüssigkeiten benötigt. Zu diesem Zweck eignen sich z. B. aliphatische oder aromatische Kohlenwasserstoffe, Oele tierischer oder pflanzlicher Herkunft und die entsprechenden denaturierten Oele (z. B. hydrierte Oele, polymerisierte Oele). Zu den bevorzugten hydrophoben organischen Flüssigkeiten gehören aliphatische Kohlenwasserstoffe wie Kerosin, Paraffin, Isoparaffin und aromatische Kohlenwasserstoffe wie Benzol, Toluol und Xylol. Handelsübliche technische Gemische von vorzugsweise verzweigten Paraffinölen mit einem Siede-Bereich von 160-260 °C, vorzugsweise 180 bis 210 °C, stehen im Vordergrund des Interesses.

Als bei der inversen Emulsionspolymerisation eingesetzte Wasser-in-Oel Emulgatoren kommen Polyoxyalkylen-, vorzugsweise Polyoxyäthylenaddukte von aliphatischen Alkoholen mit 8 bis 24 Kohlenstoffatomen wie Lauryl-, Cetyl-, Sterayl- und Oleylalkohole, von Fettsäuren mit 8 bis 24 Kohlenstoffatomen der vorstehend angegebenen Art, vorzugsweise Laurin-, Palmitin-, Stearin- und Oelsäure, von Alkylphenolen mit 8 bis 24 Kohlenstoffatomen im Alkylrest, z. B. Octyl-, Nonyl-, Dodecyl- und Dinonylphenol, und von Estern von Fettsäuren der angegebenen Art mit mehrwertigen Alkoholen, z. B. Glycerin, Pentaerythrit, Sorbit und Sorbitan in Betracht. Auch handelsübliche Gemische, d. h. Polyoxyalkylenaddukte von technischen Alkoholgemischen, Fettsäuregemischen, Alkylphenolgemischen und Estergemischen sind als Wasser-in-Oel Emulgatoren besonders geeignet. Besonders bevorzugt sind indessen Ester der Fettsäuren der angegebenen Art, bzw. Fettsäuregemische mit mehrwertigen Alkoholen der angegebenen Art, wobei Sorbit-monooleat im Vordergrund des Interesses steht.

In besonderen Fällen hat sich die Verwendung eines Emulsionsstabilisators in der Oelphase als zweckmässig erwiesen. Zu diesem Zweck eignen sich vor allem in der Oelphase lösliche Kautschuke, und zwar sowohl solche natürlicher Herkunft, z. B. Kristallgummi, als auch vorzugsweise solche synthetischer Herkunft, z. B. Polybutadien, Copolymerisate aus Styrol und Butadien und insbesondere Polyisopren, das im Vordergrund des Interesses steht.

In der Regel enthält die Oelphase etwa 2 bis 15 Gewichtsprozent Emulgator und 0 bis etwa 1, vorzugsweise 0,4 bis 0,8 Gewichtsprozent Stabilisator.

Nach dem Vermischen der Oelphase mit der wässrigen Phase, welche die Monomere der Formel (38) und gegebenenfalls die Comonomere der Formeln (39), (40) und (41) enthalten, wird in der Regel die Polymerisationsreaktion durch Zugabe eines Polymerisationsreaktion durch Zugabe eines Polymerisationsinitiators in Gang gesetzt. Als Initiatoren können die üblichen Polymerisationskatalysatoren, vorzugsweise in Form ihrer organischen oder wässrigen Lösung, eingesetzt werden, z. B. Azoverbindungen wie Azo-bis(iso-butyronitril) oder Azo-bis(dimethylvaleronitril), oxydierende Mittel, vorzugsweise Peroxyde wie Wasserstoffperoxyd oder Benzoylperoxyd oder vorzugsweise Persulfate wie Ammoniumpersulfat, ferner auch Chlorate oder Chromate, reduzierende Mittel, wie Sulfite, Bisulfite, Oxalsäure und Ascorbinsäure, sowie die Kombination, als sogenannte Redoxkatalysatoren, der vorstehend aufgeführten oxydierenden und reduzierenden Mittel. Im vorliegenden Fall eignet sich Natriumsulfit vorzugsweise als wässrige Lösung besonders gut als Initiator.

Die Polymerisationsreaktion erfolgt in der Regel bei 30 bis 90 °C, vorzugsweise 40 bis 70 °C, und verläuft exotherm, so dass die Polymerisationstemperatur gegebenenfalls durch Kühlen eingehalten werden muss.

Zur üblichen Aufarbeitung wird das erhaltene Polymerisat in der Regel durch ein vorzugsweise mit Oel und Wasser mischbares Lösungsmittel gefällt, z. B. mit Methanol, Isopropanol oder Aceton, wobei die Fällung im allgemeinen durch Zugabe der Wasser-in-Oel-Emulsion ins vorgelegte Lösungsmittel, vorzugsweise bei Raumtemperatur (15 bis 25 °C), durchgeführt wird, worauf nach der Filtration das gefällte Polymerisat vorzugsweise bei Temperaturen von höchstens 60 °C, insbesondere bei Temperaturen von etwa 30 bis 50 °C, zweckmässig unter vermindertem Druck getrocknet wird.

Die an sich bekannten, so erhaltenen (Wasser-in-Oel) Homo- oder vorzugsweise Copolymerisate werden dann als Komponente (a) nach an sich bekannten Methoden mit der Komponente (b) in wässrigem Medium vorzugsweise bei Raumtemperatur (15 bis 25 °C) und einem pH-Wert von 5 bis 9 vermischt, um zu den neuen und erfindungsgemässen Gemischen zu gelangen.

Bei ihrer Verwendung in der Kosmetikindustrie werden die erfindungsgemässen Gemische der Komponenten (a) und (b) vorzugsweise als Haarkosmetika eingesetzt.

Die erfindungsgemässen kosmetischen Mittel, vorzugsweise haarkosmetische Mittel, liegen in ihrer bevorzugten Ausführungsform als wässrige Lösungen vor, die z. B. 0,05 bis 1,5, vorzugsweise 0,2 bis 1,0 Gewichtsteile, berechnet, als Wirksubstanz, mindestens eines polymeren Ammoniumsalzes mit Strukturelementen der Formel (1), gegebenenfalls (2), (3) und/oder (4), 5 bis 20, vorzugsweise 8 bis 15, insbesondere 9 bis 12 Gewichtsteile, berechnet als Wirksubstanz, mindestens eines nicht-ionischen Tensides oder eines Tensides mit intramolekular je einer positiven und negativen Ladung als Komponente (b) und gegebenenfalls kosmetische Hilfsmittel als Komponente (c) enthalten und mit entionisiertem Wasser auf insgesamt 100 Gewichtsteile verdünnt sind.

Die fakultativen, kosmetischen Hilfsmittel als Komponente (c) sind handelsübliche Mittel, wie sie in der Haarkosmetik eingesetzt werden. In Betracht kommen z. B. Tenside, die von den als Komponente (b) eingesetzten Tensiden der angegebenen Art verschieden sind, wie Polyglycerolester und Polyglykolester von Fettsäuren, insbesondere Polyglycerololeate, im weiteren auch Polyglykole und Schaumstabilisatoren, z. B. Fettsäurepolyalkanolamide. Verdickungsmittel natürlicher oder synthetischer Herkunft wie Hydroxypropylmethylcellulose und Polyacrylsäure, Opalisierungsmittel, wie Fettsäuremonoalkanol-amide oder vorzugsweise Glycerinmonostearat, ferner auch u. a. Konservierungsmitel, Parfüms und Perlglanzmittel.

Erforderlichenfalls werden die haarkosmetischen Mittel auf einen pH-Wert von 5 bis 8, vorzugsweise 7,0 bis 7,5 und insbesondere 7,0 bis 7,2 eingestellt, was zweckmässig durch einen Zusatz von wässrigen Lösungen aus z. B. Natriumhydroxid oder Zitronensäure erreicht werden kann.

Bei der Applikation der haarkosmetischen Mittel, vorzugsweise auf menschlichem Haar, wird im Haarbehandlungsverfahren das vorstehend beschriebene, wässrige, haarkosmetische Mittel auf mit Leitungswasser angefeuchtetem Haar, in der Regel bei Raumtemperatur bis leicht erhöhter Temperatur, z. B. 20 bis 40 °C, aufgebracht und das Haar anschliessend schamponiert und konditioniert. Das so behandelte Haar kann ebenfalls in Form von Perücken oder Toupets vorliegen.

Der wesentliche Vorteil der vorliegenden Erfindung liegt darin, dass bei der Applikation der haarkosmetischen Mittel, welche als Komponente (a) die eingesetzten, polymeren, quaternären Ammoniumsalze mit Strukturelementen der Formel (1) und gegebenenfalls (2), (3) und (4) aus den entsprechenden Wasser-in-Oel Homopolymerisaten bzw. vorzugsweise Copolymerisaten mit hochmolekularen Anteilen und als Komponente (b) Tenside der angegebenen Art enthalten, eine ausgezeichnete Trocken- und vor allem Nässkämmbarkeit des behandelten Haars erzielt wird. Insbesondere ist die Nasskämmbarkeit der mit den erfindungsgemässen haarkosmetischen Mitteln behandelten Haare der Nasskämmbarkeit von Haaren, die mit herkömmlichen kosmetischen Mitteln behandelt werden, deutlich überlegen. Letztere enthalten zwar auch Mischungen aus Tensiden und Polymerisaten, jedoch weisen die Polymerisate eine andere Zusammensetzung auf, insbesondere fehlen ihnen die hochmolekularen Anteile.

In den nachfolgenden Herstellungsvorschriften und Beispielen angegebene Teile und Prozente sind Gewichtseinheiten.

Herstellungsvorschriften für die Komponente (a) (Wasser-in-Oel Emulsionscopolymerisate)

Vorschrift A :

Die folgenden drei Lösungen werden in sauerstofffreier, inerter Stickstoffatmosphäre vorbereitet :

Lösung I (Oelphase)

In einem Doppelmantelreaktionsgefäss werden

500 Teile eines verzweigten Paraffinöls (technisches Gemisch, Molekulargewicht 171, Siedebereich 188-206 °C) vorgelegt.

140 Teile einer 2,5 %igen Lösung eines synthetischen Kautschuks aus Polyisopren (Emulsionsstabilisator) in Paraffinöl der angegebenen Art und anschliessend

78 Teile Sorbitanmonooleat (Wasser-in-Oel Emulgator) werden dem vorgelegten Paraffinöl unter Rühren bei 20 °C zugegeben.

Man erhält eine klare, gelbliche Lösung.

Lösung II (wässrige Phase)

568,6 Teile Acrylamid (8 Mol) werden bei 20 °C in
700 Teilen entionisiertem, sauerstofffreiem Wasser gelöst. In diese Lösung werden
220 Teile Natriumchlorid unter Rühren eingetragen und anschliessend
1 133,2 Teile einer 50 %igen, wässrigen Lösung aus Methacryloyl-oxy-äthyl-trimethylammoniummethylsulfat (2 Mol) zugegeben.

Man erhält eine klare farblose Lösung.

Lösung III (Initiatorlösung)

0,66 Teile Natriumsulfit werden in
40 Teilen entionisiertem, sauerstofffreiem Wasser gelöst.

Copolymerisationsreaktion

Unter intensivem Rühren (3 000 U/min) wird innerhalb von 10 Minuten in inerter Stickstoffatmosphäre die Lösung II in die vorgelegte Lösung I bei 20 °C zugegeben. Man erhält eine homogene, weisse Emulsion, die bei 20 °C weitergerührt wird, bis die Viskosität einer Emulsionsprobe 14 000 m Pa.s (Brookfield Viskosimeter LV, Spindel 3, 6 U/min, 25 °C) beträgt, was im allgemeinen 10 Minuten in Anspruch nimmt. Hierauf wird das Reaktionsgemisch unter Rühren bei 300 U/min innerhalb von 30 Minuten auf 40 °C geheizt. Mittels einer Dosierpumpe wird nun die Lösung III innerhalb von 150 Minuten in das Reaktionsgemisch zugegeben, wobei die Temperatur durch Kühlen auf 40 bis 41 °C gehalten wird. Nach Beendigung der Initiatorlösung-Zugabe wird das Reaktionsgemisch bei 40 °C und 300 U/min weiter gerührt, bis die Viskosität einer Emulsionsprobe auf 7 600 mPa.s. (Brookfield Viskosimeter LV, Spindel 1, 60 U/min, 25 °C) absinkt, was im allgemeinen eine Stunde in Anspruch nimmt.

Aufarbeitung

Die erhaltene Emulsion des Copolymerisates wird unter Rühren in 24 000 Teile Aceton bei 20 °C gegeben und das Copolymerisat ausgefällt. Das ausgefällte Copolymerisat wird abfiltriert und während 2 Tagen unter vermindertem Druck bei 40°C getrocknet. Man erhält 1 100 Teile eines Copolymerisates, das in beliebiger Reihenfolge 80 Mol % Strukturelemente der Formel

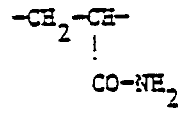 (42)

und

20 Mol % Strukturelemente der Formel

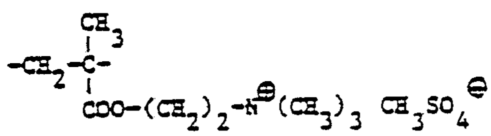 (43)

enthält, wobei das Molekulargewicht von 37 % des Copolymerisates zwischen $10^7$ und $10^9$ liegt.

Vorschrift B

Man verfährt wie in Vorschrift A angegeben, setzt jedoch in der wässrigen Phase (Lösung II) 1 108 Teile einer 40 %igen, wässrigen Lösung aus Methacryloyloxypropyl-trimethylammonium-chlorid (2 Mol) ein.

Man erhält 1 000 Teile eines Copolymerisates, das in beliebiger Reihenfolge 80 Mol % Strukturelemente der in Vorschrift A angegebenen Formel (42) und 20 Mol % Strukturelemente der Formel

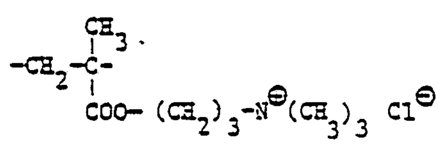 (44)

enthält, wobei das Molekulargewicht von 27 % des Copolymerisats zwischen $10^7$ und $10^9$ liegt.

Vorschrift C

Man verfährt wie in Vorschrift A angegeben, setzt jedoch in der wässrigen Phase (Lösung II) 1 471,5 Teile einer 40 %igen, wässrigen Lösung aus Methacryloyl-oxypropyl-dimethyl-n-propyl-ammonium-bromid (2 Mol) ein.

Man erhält 1 000 Teile eines Copolymerisates, das in beliebiger Reihenfolge 80 Mol % Strukturelemente der in Vorschrift A angegebenen Formel (42) und 20 Mol % Strukturelemente der Formel

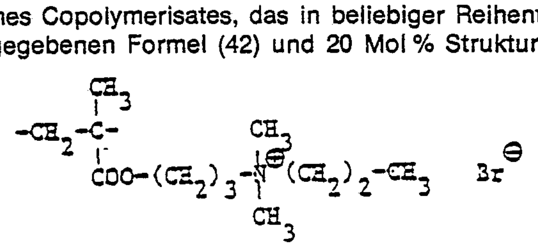 (45)

enthält, wobei das Molekulargewicht von 22 % des Copolymerisats zwischen $10^7$ und $10^9$ liegt.

### Vorschrift D

Man verfährt wie in Vorschrift A angegeben, setzt jedoch die nachfolgenden Lösungen I, II und III ein und verwendet bei der Aufarbeitung 40 000 Teile Aceton.

Lösung I

825 Teile des in Vorschrift A angegebenen Paraffinöls,
269 Teile der in Vorschrift A angegebenen 2,5 %igen Kautschuklösung und
255 Teile Sorbitanmonooleat.

Lösung II wässrige Lösung aus

681,6 Teile Acrylamid (9,6 Mol),
34,6 Teile Acrylsäure (0,48 Mol),
50,3 Teile Dimethylamino-methacrylsäure-äthylester (0,32 Mol) und
220 Teile Natriumchlorid in 700 Teilen Wasser.

Wässrige Lösung aus 1 586,5 Teilen Methacryloyl-oxyäthyl-trimethyl-ammonium-methylsulfat (5,6 Mol) in 3 100 Teilen Wasser.

Lösung III

1,07 Teile Natriumsulfit in
66,8 Teilen Wasser.

Man erhält 2 100 Teile eines Copolymerisats, das in beliebiger Reihenfolge
60 Mol % Strukturelemente der in Vorschrift A angegebenen Formel (42)
35 Mol % Strukturelemente der in Vorschrift A angegebenen Formel (43),
3 Mol % Strukturelemente der Formel

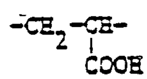 (46)

und

2 Mol % Strukturelemente der Formel

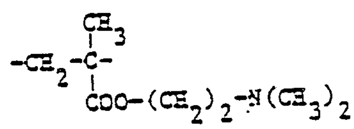 (47)

enthält, wobei das Molekulargewicht von 12 % des Copolymerisats zwischen $10^7$ und $10^9$ liegt.

### Vorschrift E

Man verfährt wie in Vorschrift A angegeben, setzt jedoch die nachfolgenden Lösungen I, II und III ein und verwendet bei der Aufarbeitung 50 000 Teile Aceton.

Lösung I*

1 628 Teile des in Vorschrift A angegebenen Paraffinöls und
191 Teile Sorbitan-monooleat

Lösung II Lösung aus

1 156,6 Teilen Acrylamid** (21, 36 Mol),
679,9 Teile Methacryloyl-oxyäthyl-trimethylammonium-methylsulfat (2,40 Mol),
37,7 Teilen Dimethylamino-methacrylsäure-äthylester** (0,24 Mol) und
600 Teilen Natriumchlorid** in
2 550 Teilen Wasser

(*enthält keine Kautschuklösung im Gegensatz zu Vorschrift A).
(**diese Komponenten werden entgegen Vorschrift A nicht separat vorgelegt).

Lösung III

1,6 Teile Natriumsulfit in
720 Teilen Wasser.

Man erhält 1 890 Teile eines Copolymerisats, das in beliebiger Reihenfolge
90 Mol % Strukturelemente der in Vorschrift A angegebenen Formel (42)
9 Mol % Strukturelemente der in Vorschrift A angegebenen Formel (43)
und
1 Mol % Strukturelemente der in Vorschrift D angegebenen Formel (47)
enthält, wobei das Molekulargewicht von 23 % des Copolymerisats zwischen $10^7$ und $10^9$ liegt.

Vorschrift F

Man verfährt wie in Vorschrift A angegeben, setzt jedoch die nachfolgenden Lösungen I, II und III ein und verwendet bei der Aufarbeitung 5 000 Teile Aceton.

Lösung I

319 Teile des in Vorschrift A angegebenen Paraffinöls
91 Teile der in Vorschrift A angegebenen 2,5 %igen Kautschuklösung und
48 Teile Sorbitanmonooleat.

Lösung II

Wässrige Lösung aus

85,3 Teilen Acrylamid (1,2 Mol)
2 720,2 Teilen einer 50 %igen, wässerigen Lösung aus Methacryloyl-oxyäthyl-trimethylammoniummethylsulfat (4,8 Mol) und
475 Teilen Wasser
(Lösung II enthält im Gegensatz zur Vorschrift A kein Natriumchlorid)

Lösung III

0,4 Teile Natriumsulfit in
25 Teilen Wasser

Man erhält 1 300 Teile eines Copolymerisates, das in beliebiger Reihenfolge
20 Mol % Strukturelemente der in Vorschrift A angegebenen Formel (42)
und
80 Mol % Strukturelemente der in Vorschrift A angegebenen Formel (43)
enthält, wobei das Molekulargewicht von 12 % des Copolymerisates zwischen $10^7$ und $10^9$ liegt.

Vorschrift G

Man verfährt wie in Vorschrift A angegeben, setzt jedoch die nachfolgenden Lösungen I, II und III ein und verwendet bei der Aufarbeitung 5 000 Teile Aceton.

Lösung I

319 Teile des in Vorschrift A angegebenen Paraffinöls
91 Teile der in Vorschrift A angegebenen 2,5 %igen Kautschuklösung
und
48 Teile Sorbitanmonooleat.

Lösung II

Wässrige Lösung aus
68 Teilen einer 50 %igen, wässrigen Lösung aus Methacryloyl-oxyäthyl-trimethylammoniummethylsulfat in
475 Teilen Wasser
(Lösung II enthält im Gegensatz zu Vorschrift A weder Acrylamid noch Natriumchlorid)

Lösung III

0,4 Teile Natriumsulfit in
25 Teilen Wasser.

Man erhält 30 Teile eines Homopolymerisates, das Strukturelemente der in Vorschrift A angegebenen Formel (43) und infolge Hydrolyse Spuren (0,001 bis 0,5 %) an Strukturelemente der in Vorschrift D angegebenen Formel (47) enthält, wobei das Molekulargewicht von 8 % des Homopolymerisates zwischen $10^7$ und $10^9$ liegt.

Herstellungsvorschrift für Lösungscopolymerisat

Vorschrift H :

Die folgenden zwei Lösungen werden in sauerstofffreier inerten Stickstoffatmosphäre vorbereitet :

Lösung I (Monomerlösung)

In einem Doppelmantelgefäss werden
71,1 Teile Acrylamid (1 Mol) und
141,7 Teile einer 50 %igen, wässrigen Lösung aus Methacryloyloxyäthyl-trimethylammonium-metylsulfat (0,25 Mol) bei 20 °C in
228 Teilen entionisiertem, sauerstoffreiem Wasser gelöst.
Man erhält eine klare farblose Lösung.

Lösung II (Initiatorlösung)

0,2 Teile Ammoniumperoxodi-sulfat werden in 150 Teilen deionisiertem sauerstofffreiem Wasser gelöst.

Copolymerisationsreaktion

Unter Rühren wird innerhalb 1 Minute in inerter Stickstoffatmosphäre die Hälfte der Lösung II in die vorgelegte Lösung I bei 35 °C zugegeben. Nach 6 Stunden wird die Reaktionslösung auf 50 °C erwärmt und die zweite Hälfte von Lösung II zugegeben. Es wird so lange gerührt, bis nach 2 bis 3 Stunden eine hochviskose Lösung entstanden ist. Ohne Rühren wird das Reaktionsgemisch stehen gelassen. Nach 24 Stunden lässt man das entstandene farblose Gel abkühlen.

Aufarbeitung

Das Gel wird zerkleinert und in 1 350 Teilen deionisiertem Wasser gelöst. Die hochviskose Lösung wird dann in 18 000 Teilen Aceton in dünnen Strang bei 20 °C eingepresst und das Copolymerisat ausgefällt. Das Copolymerisat wird dann abfiltriert und nochmals in 1 800 Teilen Aceton geknetet, bis es hart und spröde wird. Das Copolymerisat wird wieder abfiltriert und während 2 Tagen unter vermindertem Druck bei 40 °C getrocknet. Man erhält 110 Teile eines Copolymerisats, das in beliebiger Folge 80 Mol% Strukturelement der Formel

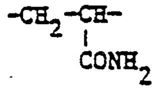

und 20 Mol%

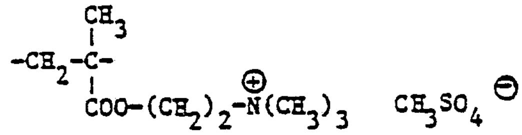

enthält, wobei das Molekulargewicht von 12 % des Copolymerisats zwischen $10^7$ und $10^9$ liegt.

Beispiel 1

1,4 Teile des polymeren Ammoniumsalzes gemäss Vorschrift A werden bei 25 bis 35 °C in kleinen Portionen in 1 000 Teile Wasser eingetragen. Es entsteht eine viskose Lösung. 20 Teile eines technischen Tensidgemisches der angegebenen Formel (28) wird in 1 000 Teilen Wasser bei 25 °C gelöst. Diese beiden Lösungen werden nun miteinander bei 25 °C vermischt. Der pH-Wert dieses Gemisches, das 0,7 %

des Ammoniumsalzes und 10 % des Tensides enthält, beträgt 6,3 und wird durch Zugabe einer 5 %igen Natriumhydroxydlösung auf 7,1 gestellt. Das als klare wässrige Lösung vorliegende, neutralisierte Gemisch wird auf eine mit Leitungswasser angefeuchtete Perücke aus ungebleichtem und ungefärbtem braunem Menschenhaar europäischer Herkunft bei 30 °C in dreimaliger Applikation im sogenannten Halbseitentest aufgebracht, worauf das Perückenhaar bei dieser Temperatur schamponiert und konditioniert wird. Im Halbseitentest wird nur die eine Hälfte der Perücke mit der vorstehend genannten Lösung schamponiert und konditioniert, während die andere Hälfte der Perücke mit einer Lösung unter gleichen Bedingungen schamponiert wird, die kein erfindungsgemässes Gemisch aus dem Ammoniumsalz und dem Tensid, sondern nur das Tensid enthält. In dieser sogenannten Leerformulierung wird die Menge an Ammoniumsalz durch die entsprechende Menge Tensid ersetzt, so dass z. B. die Leerformulierung als Vergleich 10,7 % Tensidgemisch der angegebenen Formel (28) enthält, wobei der pH-Wert der Leerformulierung ebenfalls mit der wässrigen, 30 %igen Natriumhydroxydlösung auf den pH-Wert von 7,0 gestellt wird. Nach jeder Applikation wird die Nass- und Trockenkämmbarkeit der erfindungsgemäss behandelten Perückenhälfte im Vergleich mit der mit der Leerformulierung behandelten Perückenhälfte mit Hilfe der nachfolgenden Notenskala beurteilt :

+ 3 viel besser als die Leerformulierung
+ 2 besser als die Leerformulierung
+ 1 etwas besser als die Leerformulierung
0 kein Unterschied zur Leerformulierung
− 1 etwas schlechter als die Leerformulierung
− 2 schlechter als die Leerformulierung
− 3 viel schlechter als die Leerformulierung.

Die Nasskämbarkeitsnoten betragen + 2 nach der 1. und jeweils + 3 nach der 2. und 3. Applikation, und die Trockenkämmbarkeitsnoten je + 1 nach der 1. und 2. und + (1-2) nach der 3. Applikation.

Verwendet man anstatt 1,4 Teilen des polymeren Ammoniumsalzes gemäss Vorschrift (A) 1,4 Teile des Copolymeres gemäss Vorschrift (H), so erhält man eine vergleichbare Verbesserung der Nass- und Trockenkämmbarkeit.

Beispiele 2 bis 11

Man verfährt analog Beispiel 1, wobei die eingesetzten Ammoniumsalze und Tenside (bzw. technischen Tensidgemische) zur Herstellung des erfindungsgemässen Gemisches, deren Konzentration im Gemisch, der pH-Wert des Gemisches vor dem Neutralisieren auf den pH-Wert von 7,0 bis 7,2 mit der 5 %igen Natriumhydroxydlösung oder einer wässrigen 5 %igen Zitronensäurelösung und die erhaltenen Kämmbarkeitsnoten bei der Applikation auf Menschenhaar nach dem vorstehend beschriebenen Halbseitentest in der nachfolgenden Tabelle I zusammengefasst sind.

(Siehe Tabelle I Seite 15 f.)

Tabelle I

| Beispiel Nr. | Ammonium-salz gemäss Vorschrift | Tensid der Formel | Gehalt Ammonium-salz im Gemisch % | Gehalt Tensid im Gemisch % | pH Gemisch vor Neutralisieren | Kämmbarkeitsnoten N = Nasskämmbarkeit T = Trockenkämmbarkeit nach der 1. Applikation | nach der 2. Applikation | nach der 3. Applikation |
|---|---|---|---|---|---|---|---|---|
| 2 | E | (29) | 0,7 | 10 | 6,2 | N +1<br>T +2 | N +1<br>T +2 | N + (1-2)<br>T + (1-2) |
| 3 | F | (29) | 0,7 | 10 | 6,2 | N +3<br>T +2 | N +3<br>T +2 | N + 3<br>T + 2 |
| 4 | F | (29) | 0,2 | 10 | 6,2 | N +1<br>T +1 | N +(1-2)<br>T +1 | N + (1-2)<br>T + 1 |
| 5 | G | (30) | 0,7 | 10 | 4,9 | N +1 | N +1 | N + 1 |
| 6 | G | (12) | 0,9 | 10 | 5,1 | N +(1-2)<br>T +(1-2) | N +1<br>T +1 | N + (1-2)<br>T + 1 |
| 7 | D | (10) | 0,6 | 10 | 6,5 | N +2<br>T +2 | N +2<br>T +2 | N + 2<br>T + 2 |
| 8 | B | (10) | 1,0 | 10 | 6,5 | N +1<br>T +(1-2) | N +1<br>T +1 | N + 1<br>T + 1 |
| 9 | C | (18) | 0,7 | 10 | 7,5 | N +2<br>T +1 | N +2<br>T +1 | N + (1-2)<br>T + 2 |
| 10 | E | (19) | 0,7 | 10 | 6,4 | N +1<br>T +1 | N + (1-2)<br>T + 1 | N + (1-2)<br>T + 1 |
| 11 | A | (31) | 0,7 | 10 | 5,3 | N +(1-2)<br>T +1 | N + 1<br>T + 2 | N + (1-2)<br>T + 1 |

**Patentansprüche**

1. Wässrige Gemische aus polymeren, quaternären Ammoniumsalzen und Tensiden, dadurch gekennzeichnet, dass sie

a) mindestens ein in wässrigen Tensidsystemen lösliches oder mikroemulgierbares Ammoniumsalz, das eine Molekulargewichtsverteilung von $10^4$ bis $10^9$ aufweist, wobei das Molekulargewicht von mindestens 5 Gewichtsprozent des polymeren Salzes $10^7$ bis $10^9$ beträgt und das Salz wiederkehrende Strukturelemente der Formel

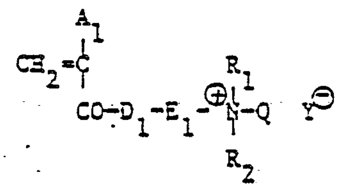 (1)

und gegebenenfalls in beliebiger Reihenfolge mindestens eines der wiederkehrenden Strukturelemente der Formeln

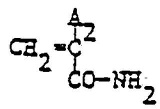 (2)

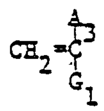 (3)

und

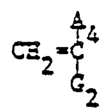 (4)

aufweist, worin $A_1$, $A_2$, $A_3$ und $A_4$ je Wasserstoff oder Methyl, $G_1$ und $G_2$ voneinander verschieden sind und je —CN, —COOH oder

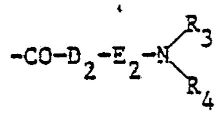

$D_1$ und $D_2$ je Sauerstoff oder —NH—, $E_1$ und $E_2$ je Alkylen mit 1 bis 4 Kohlenstoffatomen, das gegebenenfalls durch Hydroxyl substituiert ist, $R_1$, $R_2$ $R_3$ und $R_4$ je Methyl oder Aethyl, Q Alkyl, Hydroxyalkyl mit 1 bis 4 Kohlenstoffatomen oder Benzyl und $Y^{\ominus}$ ein Halogenid-, Alkylsulfat- oder Alkylphosphonatanion mit 1 bis 4 Kohlenstoffatomen im Alkylrest bedeuten, und

b) mindestens ein nicht-ionisches Tensid oder ein Tensid mit intramolekular je einer positiven und negativen Ladung unter Ausschluss von anionischen Tensiden in einem Gewichtsverhältnis a) : b) von (1) : (2 bis 400) enthalten.

2. Gemische nach Anspruch 1, dadurch gekennzeichnet, dass sie als Komponente a) ein Ammoniumsalz enthalten, das durch Wasser-in-Oel Emulsionspolymerisation oder Lösungspolymerisation eines quaternären Ammoniumsalzes der Acrylsäurereihe und gegebenenfalls mindestens eines weiteren Monomeren auf Acrylbasis erhältlich ist.

3. Gemische nach einem der Ansprüche 1 oder 2, dadurch gekennzeichnet, dass sie als Komponente a) ein Ammoniumsalz enthalten, das durchschnittlich 5 bis 100 Mol-% Strukturelemente der Formel

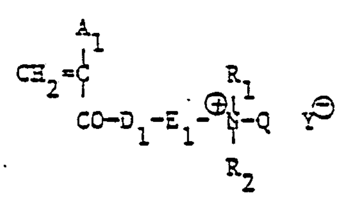 (1)

0 bis durchschnittlich 95 Mol-% Strukturelemente der Formel

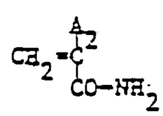 (2)

und

0 bis durchschnittlich 10 Mol-% Strukturelemente der Formel

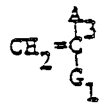 (3) und gegebenenfalls 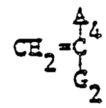 (4)

aufweisen, worin $A_1$, $A_2$, $A_3$, $A_4$, $D_1$, $E_1$, $G_1$, $G_2$, $R_1$, $R_2$, Q und $Y^{\ominus}$ die in Anspruch 1 angegebene Bedeutung haben.

4. Gemische nach einem der Ansprüche 1 bis 3, dadurch gekennzeichnet, dass sie als Komponente a) ein Ammoniumsalz mit einer Molekulargewichtsverteilung von $10^4$ bis $10^9$ enthalten, wobei das Molekulargewicht von 5 bis 60 Gewichtsprozent des polymeren Salzes $10^7$ bis $10^9$ beträgt.

5. Gemisch nach Anspruch 4, dadurch gekennzeichnet, dass das Molekulargewicht von 20 bis 50 Gewichtsprozent des als Komponente a) eingesetzten polymeren Salzes $10^7$ bis $10^9$ beträgt.

6. Gemische nach einem der Ansprüche 1 bis 5, dadurch gekennzeichnet, dass sie als nicht-ionisches Tensid für die Komponente b) äthoxylierte Fettsäuren, Fettalkohole, Fettsäureamide, Alkylphenole oder Kohlenhydrate, Addukte aus Aethylen- und Propylenoxid, Phosphorsäurepolyglykolester oder Aminoxide enthalten.

7. Gemische nach einem der Ansprüche 1 bis 5, dadurch gekennzeichnet, dass sie als Tensid mit intramolekular je einer positiven und negativen Ladung für die Komponente b) Betaine oder Sulfobetaine enthalten, die sich von einem Imidazolinderivat oder einem offenkettigen, aliphatischen Amin ableiten.

8. Verfahren zur Herstellung der Gemische gemäss einem der Ansprüche 1 bis 7, wobei man ein Monomer der Formel

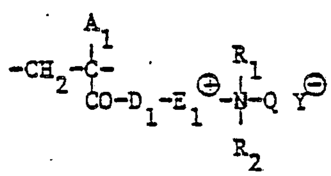 (38)

und gegebenenfalls mindestens ein Comonomer einer der Formeln

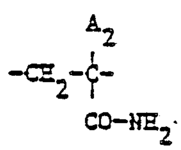 (39)

und 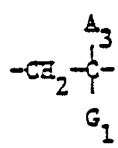 (40)

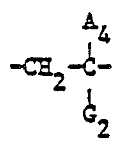 (41)

worin $A_1$, $A_2$, $A_3$, $A_4$, $D_1$, $E_1$, $G_1$, $G_2$, $R_1$, $R_2$, Q und $Y^{\ominus}$ die in Anspruch 1, angegebenen Bedeutungen haben, durch Wasser-in-Oel Emulsionspolymerisation in Gegenwart eines Wasser-in-Oel Emulgators und gegebenenfalls eines Emulsionsstabilisators oder durch Lösungspolymerisation, jeweils in Gegenwart eines Polymerisationsinitiators zur Komponente a) polymerisiert, das Polymerisat mit einem sowohl in Wasser wie in Oel löslichen Lösungsmittel ausfällt und anschliessend trocknet, dadurch gekennzeichnet, dass man das so erhaltene Polymerisat als Komponente a) einsetzt und mit mindestens einem nicht-ionischen Tensid oder einem Tensid mit intramolekular je einer positiven und negativen Ladung als Komponente b) in wässrigem Medium bei Raumtemperatur und einem pH-Wert von 5 bis 9 vermischt.

9. Verwendung der Gemische aus Ammoniumsalzen und Tensiden gemäss einem der Ansprüche 1 bis 7, in kosmetischen Mitteln.

10. Verwendung nach Anspruch 9, dadurch gekennzeichnet, dass die Gemische in haarkosmetischen Mitteln eingesetzt werden.

11. Kosmetische Mittel, dadurch gekennzeichnet, dass sie mindestens ein Gemisch aus Ammoniumsalzen und Tensiden gemäss einem der Ansprüche 1 bis 7 enthalten.

12. Kosmetische Mittel nach Anspruch 11, dadurch gekennzeichnet, dass sie als Haarkosmetika einsetzbar sind.

13. Haarkosmetische Mittel nach Anspruch 12, dadurch gekennzeichnet, dass sie 0,05 bis 1,5 Gewichtsteile, berechnet als Wirksubstanz, mindestens eines Ammoniumsalzes als Komponente a) 5 bis

20 Gewichtsteile, berechnet als Wirksubstanz, mindestens eines nicht-ionischen Tensides oder eines Tensides mit intramolekular je einer positiven und negativen Ladung als Komponente b) und gegebenenfalls kosmetische Hilfsmittel als Komponente c) enthalten und mit entionisiertem Wasser auf insgesamt 100 Gewichtsteile verdünnt sind.

14. Haarkosmetische Mittel nach Anspruch 12, dadurch gekennzeichnet, dass sie mit einer wässrigen Lösung aus Natriumhydroxid oder Zitronensäure auf einen pH-Wert von 5 bis 8 eingestellt sind.

15. Haarbehandlungsverfahren, dadurch gekennzeichnet, dass man ein kosmetisches Mittel nach einem der Ansprüche 11 bis 14 auf mit Leitungswasser angefeuchtetem Haar bei 20 bis 40 °C aufbringt, das Haar schamponiert und konditioniert.

16. Das nach dem Verfahren gemäss Anspruch 15 behandelte Haar, das in Form von Perücken oder Toupets vorliegt.

**Claims**

1. An aqueous mixture of a polymeric, quaternary ammonium salt and a surfactant, which mixture contains

a) at least one ammonium salt which is soluble in or forms a microemulsion in aqueous surfactant systems and which has a molecular weight distribution of $10^4$ to $10^9$, the molecular weight of at least 5 per cent by weight of the polymeric salt being $10^7$ to $10^9$ and the salt containing recurring structural elements of the formula

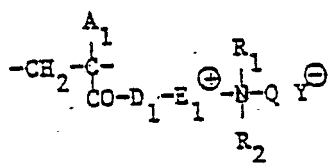

(1)

and optionally, in any order, at least one of the recurring structural elements of the formulae

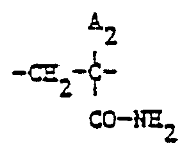

(2)

and

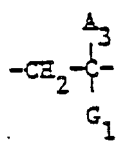

(3)

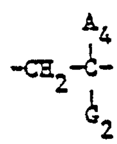

(4)

in which each of $A_1$, $A_2$, $A_3$ and $A_4$ is hydrogen or methyl, $G_1$ and $G_2$ are different and are each —CN, —COOH or

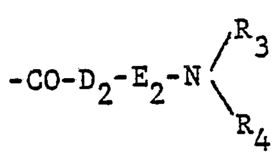

each of $D_1$ and $D_2$ is oxygen or —NH—,

each of $E_1$ and $E_2$ is alkylene having 1 to 4 carbon atoms which is unsubstituted or substituted by hydroxy, each of $R_1$, $R_2$, $R_3$ and $R_4$ is methyl or ethyl, Q is alkyl, hydroxy-alkyl having 1 to 4 carbon atoms or benzyl, and $Y^{\ominus}$ is a halide, alkylsulfate or alkylphosphonate anion having 1 to 4 carbon atoms in the alkyl moiety, and

b) at least one non-ionic surfactant or a surfactant containing one positive and one negative charge in the molecule, excluding anionic surfactants, in a weight ratio of (a) : (b) of (1) : (2 to 400).

2. A mixture according to claim 1, which contains, as component (a), an ammonium salt which can be obtained by water-in-oil emulsion polymerisation or solution polymerisation of a quaternary ammonium salt of the acrylic acid series and optionally at least one other acrylic monomer.

3. A mixture according to either claim 1 or 2, which contains, as component (a), an ammonium salt which has on average 5 to 100 mol% of structural elements of the formula

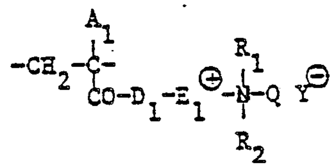 (1)

0 to on average 95 mol% of structural elements of the formula

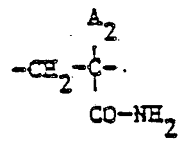 (2)

and 0 to on average 10 mol% of structural elements of the formula

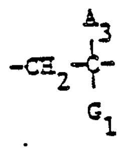 (3) and optionally 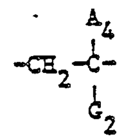 (4)

in which $A_1$, $A_2$, $A_3$, $A_4$, $D_1$, $E_1$, $G_1$, $G_2$, $R_1$, $R_2$, Q and $Y^{\ominus}$ are as defined in claim 1.

4. A mixture according to any one of claims 1 to 3, which contains, as component (a), an ammonium salt with a molecular weight distribution of $10^4$ to $10^9$, the molecular weight of 5 to 60 per cent by weight of the polymeric salt being $10^7$ to $10^9$.

5. A mixture according to claim 4, in which the molecular weight of 20 to 50 per cent by weight of the polymeric salt used as component (a) is $10^7$ to $10^9$.

6. A mixture according to any one of claims 1 to 5, which contains, as the non-ionic surfactant for component (b), an ethoxylated fatty acid, fatty alcohol, fatty acid amide, alkylphenol or carbohydrate, an adduct of ethylene oxide and propylene oxide, a phosphoric acid polyglycol ester or an amine oxide.

7. A mixture according to any one of claims 1 to 5, which contains, as the surfactant with one positive and one negative charge in the molecule for component (b), a betaine or sulfobetaine which is derived from an imidazoline derivative or an open chain, aliphatic amine.

8. A process for the preparation of a mixture according to any one of claims 1 to 7, in which a monomer of the formula

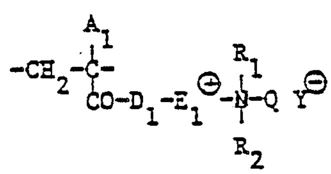 (38)

and optionally at least one comonomer of one of the formulae

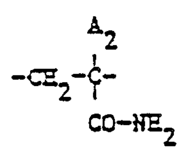 (39)

and 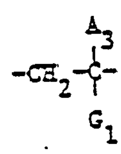 (40)

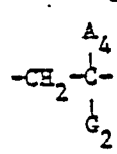 (41)

in which $A_1$, $A_2$, $A_3$, $A_4$, $D_1$, $E_1$, $G_1$, $G_2$, $R_1$, $R_2$, Q and $Y^{\ominus}$ are as defined in claim 1, are copolymerised by water-in-oil emulsion polymerisation in the presence of a water-in-oil emulsifier and optionally an

emulsion stabiliser, or by solution polymerisation, in each case in the presence of a polymerisation initiator, to give component (a), and the polymer is precipitated with a solvent which is soluble both in water and in oil, and then dried, which process comprises using the resultant polymer as component (a) and mixing this with at least one non-ionic surfactant or a surfactant which has one positive and one negative charge in the molecule, as component (b), at room temperature and at a pH value of 5 to 9.

9. Use of a mixture of an ammonium salt and a surfactant according to any one of claims 1 to 7 in a cosmetic.

10. Use according to claim 9, which comprises using the mixture in a hair cosmetic.

11. A cosmetic, which contains at least one mixture of an ammonium salt and a surfactant according to any one of claims 1 to 7.

12. A cosmetic according to claim 11, which can be used as a hair cosmetic.

13. A hair cosmetic according to claim 12, which contains 0.05 to 1.5 parts by weight, calculated as active ingredient, of at least one ammonium salt as component (a), 5 to 20 parts by weight, calculated as active ingredient, of at least one non-ionic surfactant or a surfactant which has one positive and one negative charge in the molecule as component (b), and optionally a cosmetic auxiliary as component (c), and which is diluted to a total of 100 parts by weight with demineralised water.

14. A hair cosmetic according to claim 12, which is adjusted to a pH value of 5 to 8 with an aqueous solution of sodium hydroxide or citric acid.

15. A hair treatment method, which comprises applying a cosmetic according to any one of claims 11 to 14 to hair, which has been damped with tapwater, at 20 to 40 °C and shampooing and conditioning the hair.

16. Hair which has been treated by the method according to claim 15 and is in the form of a wig or toupee.

**Revendications**

1. Mélanges aqueux de sels d'ammonium quaternaire polymères et d'agents tensio-actifs caractérisés par le fait qu'ils contiennent

a) au moins un sel d'ammonium soluble ou micro-émulsionnable dans des systèmes tensio-actifs aqueux, qui présente une distribution de poids moléculaire allant de $10^4$ à $10^9$, le poids moléculaire d'au moins 5 % en poids du sel polymère allant de $10^7$ à $10^9$, et qui présente des éléments structuraux répétitifs de formule

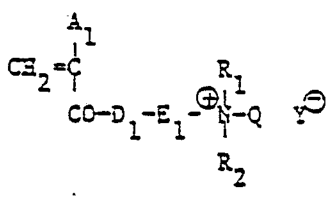
(1)

et éventuellement, en ordre quelconque, au moins un des éléments structuraux répétitifs de formules

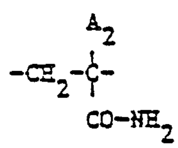
(2)

et

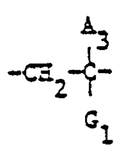
(3)

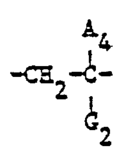
(4)

dans lesquelles $A_1$, $A_2$, $A_3$ et $A_4$ sont chacun un atome d'hydrogène ou un groupe méthyle ; $G_1$ et $G_2$ sont différents l'un de l'autre et représentent chacun —CN, —COOH ou

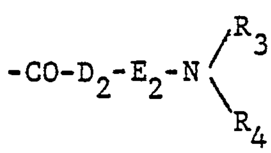

$D_1$ et $D_2$ représentent chacun un atome d'oxygène ou —NH—; $E_1$ et $E_2$ représentent chacun un groupe alkylène ayant de 1 à 4 atomes de carbone qui est éventuellement substitué par un radical hydroxyle ; $R_1$, $R_2$, $R_3$ et $R_4$ représentent chacun un groupe méthyle ou éthyle ; Q représente un groupe alkyle, hydroxy-alkyle ayant de 1 à 4 atomes de carbone ou benzyle ; et $Y^{\ominus}$ représente un anion halogénure, alkylsulfate ou alkylphosphonate contenant de 1 à 4 atomes de carbone dans le résidu alkyle ; et

b) au moins un agent tensio-actif non ionique ou un agent tensio-actif ayant intramoléculairement à la fois une charge positive et une charge négative, à l'exclusion d'agents tensio-actifs anioniques, en un rapport de poids de (a) : (b) de (1) : (2 à 400).

2. Mélanges selon la revendication 1, caractérisés par le fait qu'ils contiennent en tant que composant (a) un sel d'ammonium qui est préparable par polymérisation en solution ou polymérisation en. émulsion eau-dans-huile d'un sel d'ammonium quaternaire de la série de l'acide acrylique et éventuellement d'au moins un autre monomère à base acrylique.

3. Mélanges selon l'une des revendications 1 et 2, caractérisés par le fait qu'ils contiennent en tant que composant (a) un sel d'ammonium qui présente en moyenne de 5 à 100 % en moles d'éléments structuraux de formule

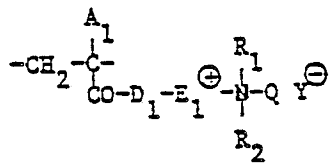 (1)

de 0 à en moyenne 95 % en moles d'éléments structuraux de formule

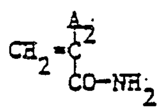 (2)

et de 0 à en moyenne 10 % en moles d'éléments structuraux de formule

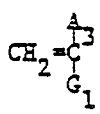 (3) et éventuellement 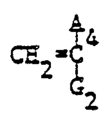 (4),

dans lesquelles $A_1$, $A_2$, $A_3$, $A_4$, $D_1$, $E_1$, $G_1$, $G_2$, $R_1$, $R_2$, Q et $Y^{\ominus}$ ont les significations données dans la revendication 1.

4. Mélanges selon l'une des revendications 1 à 3, caractérisés par le fait qu'ils contiennent en tant que composant (a) un sel d'ammonium ayant une distribution de poids moléculaire allant de $10^4$ à $10^9$, le poids moléculaire de 5 à 60 % en poids du sel polymère allant de $10^7$ à $10^9$.

5. Mélange selon la revendication 4, caractérisé par le fait que le poids moléculaire de 20 à 50 % en poids du sel polymère introduit en tant que composant (a) va de $10^7$ à $10^9$.

6. Mélanges selon l'une des revendications 1 à 5, caractérisés par le fait qu'ils contiennent en tant qu'agent tensio-actif non ionique pour le composant (b) des acides gras, des alcools gras, des amides d'acides gras, des alkylphénols ou des hydrates de carbone oxy-éthylés, des produits d'addition d'oxyde d'éthylène et d'oxyde de propylène, des phosphates de polyglycols ou des oxydes d'amines.

7. Mélanges selon l'une des revendications 1 à 5, caractérisés par le fait qu'ils contiennent pour le composant (b), en tant qu'agent tensio-actif ayant intramoléculairement à la fois une charge positive et une charge négative, des bétaïnes ou des sulfobétaïnes qui dérivent d'un dérivé d'imidazoline ou d'une amine aliphatique à chaîne ouverte.

8. Procédé pour la préparation des mélanges selon l'une des revendications 1 à 7, dans lequel on polymérise un monomère de formule

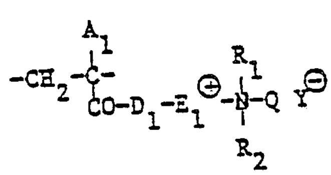 (38)

et éventuellement au moins un comonomère d'une des formules

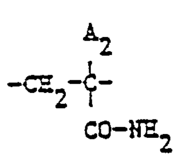 (39)

et 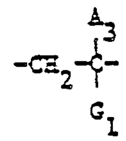 (40)

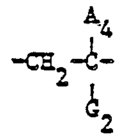 (41)

dans lesquelles $A_1$, $A_2$, $A_3$, $A_4$, $D_1$, $E_1$, $G_1$, $G_2$, $R_1$, $R_2$, Q et $Y^{\ominus}$ ont les significations données dans la revendication 1, par polymérisation en émulsion eau-dans-huile en présence d'un émulsifiant eau-dans-huile et éventuellement d'un stabilisant d'émulsion, ou par polymérisation en solution, chaque fois en présence d'un amorceur de polymérisation, pour aboutir au composant (a), on fait précipiter le polymère avec un solvant soluble aussi bien dans l'eau que dans l'huile, puis on le sèche, caractérisé par le fait que l'on introduit en tant que composant (a) le polymère ainsi obtenu et qu'on le mélange avec, en tant que composant (b), au moins un agent tensio-actif non ionique ou un agent tensio-actif ayant intramoléculairement à la fois une charge positive et une charge négative, en milieu aqueux, à la température ambiante et à un pH d'une valeur comprise entre 5 et 9.

9. Utilisation des mélanges de sels d'ammonium et d'agents tensio-actifs selon l'une des revendications 1 à 7 dans des produits cosmétiques.

10. Utilisation selon la revendication 9, caractérisée par le fait que les mélanges sont introduits dans des produits cosmétiques capillaires.

11. Produits cosmétiques, caractérisés par le fait qu'ils contiennent au moins un mélange de sels d'ammonium et d'agents tensio-actifs selon l'une des revendications 1 à 7.

12. Produits cosmétiques selon la revendication 11, caractérisés par le fait qu'ils sont utilisables en tant que cosmétiques capillaires.

13. Produits cosmétiques capillaires selon la revendication 12, caractérisés par le fait qu'ils contiennent de 0,05 à 1,5 partie en poids, calculée en tant que substance active, d'au moins un sel d'ammonium en tant que composant (a), de 5 à 20 parties en poids, calculées en tant que substance active, d'au moins un agent tensio-actif non ionique ou d'un agent tensio-actif ayant intramoléculairement à la fois une charge positive et une charge négative en tant que composant (b), et éventuellement des produits cosmétiques auxiliaires en tant que composant (c), et qu'ils sont dilués à un total de 100 parties en poids avec de l'eau désionisée.

14. Produits cosmétiques capillaires selon la revendication 12, caractérisés par le fait qu'ils sont ajustés à un pH d'une valeur comprise entre 5 et 8 avec une solution aqueuse d'hydroxyde de sodium ou d'acide citrique.

15. Procédé de traitement capillaire, caractérisé par le fait que l'on applique à une température allant de 20 à 40 °C un produit cosmétique selon l'une des revendications 11 à 14 sur des cheveux mouillés à l'eau du robinet, que l'on shampooigne les cheveux et qu'on les conditionne.

16. Les cheveux traités suivant le procédé selon la revendication 15, qui se trouvent sous forme de perruques ou de postiches.